(19) Europäisches Patentamt
European Patent Office
Office européen des brevets

(11) Veröffentlichungsnummer: **0 002 007**
**B1**

# (12) EUROPÄISCHE PATENTSCHRIFT

(45) Veröffentlichungstag der Patentschrift: **28.07.82**

(21) Anmeldenummer: **78101291.9**

(22) Anmeldetag: **02.11.78**

(51) Int. Cl.³: **C 08 K 5/34**, C 08 K 5/00, C 08 L 27/04, C 07 D 211/90

## (54) Neue Pyridindicarbonsäureesterderivate und ihre Gemische mit metallhaltigen Stabilisatoren sowie ihre Verwendung zur Stabilisierung von chlorhaltigen Thermoplasten.

(30) Priorität: **11.11.77 CH 13799/77**

(43) Veröffentlichungstag der Anmeldung: **30.05.79 Patentblatt 79/11**

(45) Bekanntmachung des Hinweises auf die Patenterteilung: **28.07.82 Patentblatt 82/30**

(84) Benannte Vertragsstaaten: **BE CH DE FR GB NL SE**

(56) Entgegenhaltungen:
DE - A - 2 349 538
DE - A - 2 534 744
FR - A - 2 239 496
US - A - 3 857 849

(73) Patentinhaber: **CIBA-GEIGY AG**
**Patentabteilung Postfach**
**CH-4002 Basel (CH)**

(72) Erfinder: **Abeler, Gerd, Dr.**
**Wilhelm-Leuschner Strasse 235**
**D-6103 Griesheim über Darmstadt (DE)**
Erfinder: **Maul, Rudolf, Dr.**
**Chrodegangstrasse 6a**
**D-6143 Lorsch/Hessen (DE)**

**EP 0 002 007 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Neue Pyridindicarbonsäureesterderivate und ihre Gemische mit metallhaltigen Stabilisatoren sowie ihre Verwendung zur Stabilisierung von chlorhaltigen Thermoplasten

Die vorliegende Erfindung betrifft neue Pyridindicarbonsäureesterderivate sowie die Verwendung von Pyridindicarbonsäureesterderivaten zusammen mit Synergisten zur Stabilisierung von chlorhaltigen Thermoplasten.

Es ist bekannt, chlorhaltigen Thermoplasten, zum Beispiel Polyvinylchlorid, Stabilisatoren zuzusetzen, um durch thermische Belastung hervorgerufene Schädigungen und/oder Verfärbungen im Substrat zurückzudrängen.

Zu den wirksamen bekannten Thermostabilisatoren zählen beispielsweise organische Metallverbindungen von Magnesium, Calcium, Strontium, Barium, Zink, Cadmium, Zinn, Blei und Antimon. Auch Mischungen einiger dieser Verbindungen sind bekannt.

Es sind aber auch zahlreiche metallfreie Thermostabilisatoren für chlorhaltige Thermoplaste in der Patentliteratur erwähnt. Nur wenige Produkte jedoch entsprechen den technischen Erfordernissen und finden tatsächlich Verwendung, wie beispielsweise die Aminocrotonsäureester, Diphenylthioharnstoff und $\alpha$-Phenylindol. Aber auch diese haben noch störende Eigenschaften, wie mangelnde Eigenstabilität und Zersetzungsneigung bei Verarbeitungstemperatur und/oder hohe Flüchtigkeit und/oder geringe Substratverträglichkeit und/oder mangelnde Lichtstabilität.

Aus der DT—OS 2 436 007 sind aber gute beständige Stabilisatoren bekannt, welche die obengenannten Nachteile nicht aufweisen. Es handelt sich dabei um Verbindungen der Formel

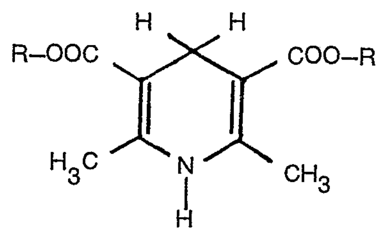

worin R ein Kohlenwasserstoffradikal, beispielsweise ein Alkyl-, Cycloalkyl- oder Phenylradikal bedeutet. Diese Stabilsatoren vermitteln wohl eine gute Kurzzeitstabilisierung, sie sind jedoch etwas schwächer in Bezug auf Langzeitstabilisierung.

Es wurde nun gefunden, dass die gleichzeitige Verwendung eines Diesters der 1,4-Dihydro-2,6-dimethylpyridin-3,5-dicarbonsäure und eines ein Metall aus der Gruppe Barium, Cadmium, Zinn oder Antimon oder ferner auch Zink kombiniert mit mindestens einem der vorgenannten Metalle enthaltenden Stabilisators eine überraschend starke synergistische Wirkung, d.h. eine weitaus stärkere als die gegebenenfalls zu erwartende additive thermostabilisierende Wirkung hat. Gegenstand der vorliegenden Erfindung ist demnach ein Stoffgemisch bestehend aus

a) einem Dicarbonsäurediester der Formal (I)

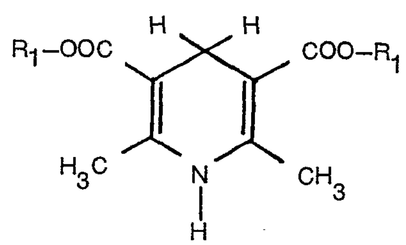

(I)

worin $R_1$ verzweigtes oder unverzweigtes $C_1$—$C_{20}$ Alkyl, $C_5$—$C_7$ Cycloalkyl, Phenyl oder eine Gruppe der Formel —$(Ch_2)_p$—X—R ist, worin X Schwefel oder Sauerstoff, R verzweigtes oder unverzweigtes $C_1$—$C_{20}$ Alkyl und

p die Zahlen 1—8 bedeuten, und

b) einem ein Metall aus der Gruppe Barium, Cadmium, Zinn oder Antimon oder ferner auch Zink kombiniert mit mindestens einen der vorgenannten Metallen enthaltenden Stabilisator.

R und $R_1$ bedeuten als $C_1$—$C_{20}$ Alkyl lineares oder verzweigtes, insbesondere lineares $C_1$—$C_{12}$ Alkyl. Beispiele sind Methyl, Aethyl, Propyl, Isopropyl, Butyl, tert.-Butyl, n-Pentyl, $\alpha$-Methylpentyl, Hexyl, 2,4-Dimethylpentyl, Octyl, 6-Methylheptyl, 2-Aethylhexyl, Decyl, Dodecyl, Octadecyl, Octadecyläthyl, Eicosyl. $R_1$ ist als Alkyl bevorzugt $C_1$—$C_4$ Alkyl, insbesondere Aethyl sowie vor allem auch Dodecyl und R ist bevorzugt Dodecyl.

$R_1$ bedeutet als $C_5$—$C_7$ Cycloalkyl Cyclopentyl, Cycloheptyl und insbesondere Cyclohexyl.

Für das synergistische Stoffgemisch werden Dicarbonsäurediester der Formel (I) bevorzugt, worin $R_1$ eine Gruppe —$(CH_2)_p$—X—R mit der oben angegebenen Bedeutung und insbesondere eine Gruppe —$(CH_2)_p$—S—$(CH_2)_q$—$CH_3$ ist, worin p die Zahlen 1—4, insbesondere 2 und 3 und q die Zahlen

1—11, insbesondere 11 bedeuten.

Als metallhaltige Stabilisatoren werden Ba/Zn-, Ba/Cd/Zn- und bevorzugt Organozinn-Stabilisatoren verwendet. Es handelt sich daher insbesondere um Mischungen aus Ba- und Zn- oder Ba-, Zn- und Cd-Stearaten oder Carboxylaten, bzw. um Organozinn-Mercaptiden oder Maleaten wie sie beispielsweise in Pure & Appl. Chem., Vol. 49, S. 627—648, Pergamon Press, 1977 (H. O. Wirth, H. Andreas, The Stabilization of PVC against Heat and Light) beschrieben sind.

1,4-Dihydro-2,6-dimethylpyridin-3,5-dicarbonsäurediester der Formel (I), worin R eine Gruppe der Formel $-(CH_2)_p-S-R$ bedeutet sind neu und überraschenderweise wirksamer als die vorbekannten Diester der Formel (I).

Eine weiterer Gegenstand der vorliegenden Erfindung sind demnach die Verbindungen der Formel (II)

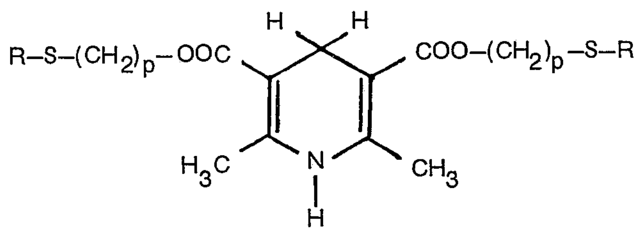

(II)

worin R verzweigtes oder unverzweigtes $C_1$—$C_{20}$ Alkyl und p die Zahlen 1—8 bedeuten. Bevorzugt sind Verbindungen der Formel (III)

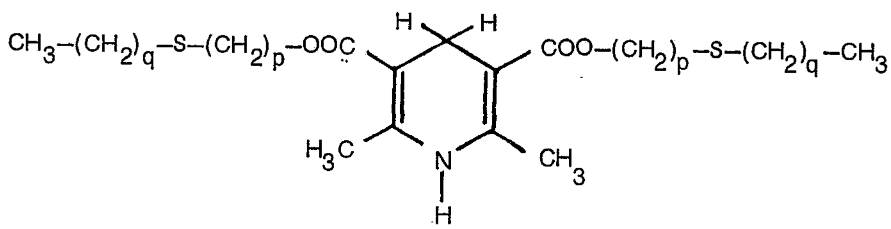

(III)

worin p die Zahlen 1—4, insbesondere 2 und 3 und q die Zahlen 1—11, insbesondere 11 bedeuten.

Die erfindungsgemäss zu verwendenden metallhaltigen Stabilisatoren sind allgemein bekannt und werden bereits verschiedentlich zur Stabilisierung von chlorhaltigen Thermoplasten eingesetzt.

Die neuen 1,4-Dihydro-2,6-dimethylpyridin-3,5-dicarbonsäurediester der Formel (II) können sowohl nach einem neuen Verfahren gemäss folgender Gleichung

$$\frac{2}{n}(CH_2O)_n + 4CH_3COCH_2COO-(CH_2)_p-S-R + NH_2COO^{-}\,{}^{+}NH_4 =$$

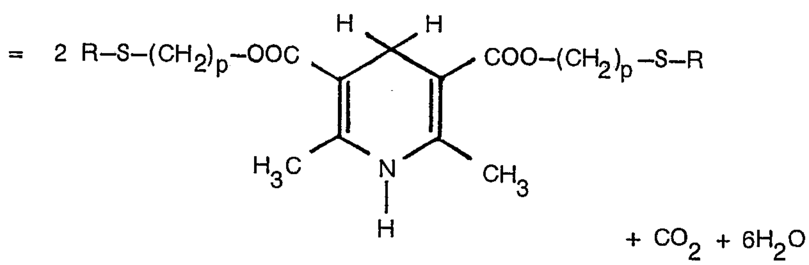

+ $CO_2$ + $6H_2O$

als auch in Analogie zu bereits bekannten Verfahren hergestellt werden, wie beispielsweise

a) das Verfahren von Hantzsch (Org. Synth., Coll. Vol. II, 214 und 215), bei dem man Acetessigester mit Formaldehyd und Ammoniak reagieren lässt, oder

b) die Umsetzung von Urotropin mit Ammoniumacetat (GB—PS 1 294 650).

Das Verfahren a) ist zweistufig und erfordert relativ lange Reaktionszeiten, während Verfahren b) dem neuen oben erwähnten ähnlich ist, wobei beide einstufig sind und nur kurze Reaktionszeiten erfordern.

Verbindungen der Formel (I), worin $R_1$ Alkyl, Cycloalkyl oder Phenyl bedeutet sind bereits aus der DT—OS 2 436 007 bekannt. Sie können alle nach einem der drei oben genannten Verfahren hergestellt werden.

Das erfindungsgemässe synergistische Stoffgemisch eignet sich hervorragend zur Stabilisierung von chlorhaltigen Thermoplasten gegen den Abbau durch Wärmeeinwirkung.

Ein weiterer Gegenstand vorliegender Erfindung ist daher eine stabilisierte Zusammensetzung enthaltend ein chlorhaltiges thermoplastisches Polymer und als Stabilisator ein Stoffgemisch aus 0,5—2,5, bevorzugt 1—2 Gew.% des Dicarbonsäurediesters der Formel (I) und 0,05—1,5, bevorzugt 0,2—0,5 Gew.% des metallhaltigen Stabilisators, bezogen auf die gesamte Zusammensetzung.

Die Einzelbestandteile des Stoffgemisches werden einzeln oder bereits vermischt den zu stabilisierenden Thermoplasten vor der Verarbeitung in üblichen Einrichtungen einverleibt.

Als chlorhaltige Thermoplaste seien genannt: Polyvinylidenchlorid und bevorzugt Polymere aus oder auf der Grundlage von Vinylchlorid. Bevorzugt sind Suspensions- und Massepolymere und ausgewaschene, also emulgatorarme Emulsionspolymere. Beim Polyvinylchlorid kann es sich um weichmacherhaltiges oder um Hart-PVC handeln.

Als Comonomere für Thermoplaste auf der Grundlage von Vinylchlorid seien genannt: Vinilidenchlorid, Transdichloräthen, Aethylen, Propylen, Butylen, Maleinsäure, Acrylsäure, Fumarsäure oder Itaconsäure.

Vor, während oder nach der Zugabe des erfindungsgemässen Stabilisatorgemisches können je nach Verwendungszweck der Formmasse weitere Zusätze einverleibt werden, wie Gleitmittel, bevorzugt Montanwachse oder Glycerinester, Füllstoffe, verstärkende Füllstoffe wie Glasfasern, Modifikatoren wie etwa Schlagzähzusätze und/oder Pigmente.

Ein bevorzugter Zusatz sind Lichtschutzmittel, insbesondere UV-Stabilisatoren, die im allgemeinen in Mengen von 0,001 bis 2 Gew.%, bezogen auf die gesamte Zusammensetzung zugegeben werden. Geeignete Lichtschutzmittel und UV-Stabilisatoren sind beispielsweise:

*2-(2'-Hydroxyphenyl)-benztriazole,* wie z.B. das 5'Methyl-3',5'-Di-tert.butyl-, 5'-Tert-butyl-, 5'-(1,1,3,3,-Tetramethylbutyl)-, 5-Chlor-3',5'-di-tert.butyl-, 5-Chlor-3'-tert-butyl-5'-methyl-, 3'-sec.-Butyl-5'-tert.butyl-, 3'-$\alpha$-Methylbenzyl-5'-methyl-, 3'-$\alpha$-Methylbenzyl-5'-methyl-5-chlor-, 4'-Hydroxy-, 4'-Methoxy-, 4'-Octoxy-, 3',5'-Di-tert.amyl-, 3'-Methyl-5'-carbomethoxyäthyl-, 5-Chlor-3', 5'-di-tert.amyl-Derivat.

*2,4-Bis-(2'-hydroxyphenyl)-6-alkyl-s-triazine,* wie z.B. 6-Aethyl-, 6-Heptadecyl-, 6-Undecyl-Derivat.

*2-Hydroxybenzophenone,* wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy- Derivat.

*1,3-Bis-(2'-hydroxybenzoyl)-benzole,* wie z.B. 1,3-Bis-(2'-hydroxy-4'-hexyloxy-benzoyl)-benzol, 1,3-Bis-(2'-hydroxy-4'-octyloxy-benzoyl)-benzol, 1,3-Bis-(2'-hydroxy-4'-dodecyloxy-benzoyl)-benzol.

*Ester von gegebenenfalls substituierten Benzoesäuren,* wie z.B. Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester oder -octadecylester oder -2-methyl-4,6-di-tert.butylphenylester.

*Acrylate,* wie z.B. $\alpha$-Cyan-$\beta$, $\beta$-diphenylacrylsäure-äthylester bzw. -isooctylester, $\alpha$-Carbomethoxy-zimtsäuremethylester, $\alpha$-Cano-$\beta$-methyl-p-methoxy-zimtsäuremethylester bzw. -butylester, N-($\beta$-Carbomethoxyvinyl)-2-methylindolin.

*Nickelverbindungen,* wie z.B. Nickelkomplexe de 2,2'-Thio-bis[4-(1,1,3,3-tetramethylbutyl)-phenol], wie der 1:1- oder der 1:2-Komplex, gegebenenfalls mit zusätzlichen Liganden wie n-Butylamin, Triäthanolamin oder N-Cyclohexyl-di-äthanolamin, Nickelkomplexe des Bis[2-hydroxy-4-(1,1,3,3-tetramethylbutyl)-phenyl]-sulfons, wie der 2:1-Komplex, gegebenenfalls mit zusätzlichen Liganden wie 2-Aethylcarbonsäure, Nickeldibutyldithiocarbamat, Nickelsalze von 4-Hydroxy-3,5-di-tert.butylbenzyl-phosphonsäure-monoalkylestern wie vom Methyl-, Aethyl- oder Butylester, Nickelkomplexe von Ketoximen wie von 2-Hydroxy-4-methylphenyl-undecylketonoxim, Nickel-3,5-di-tert.butyl-4-hydroxybenzoat, Nickelisopropylxanthogenat.

*Sterisch gehinderte Amine,* wie z.B. 4-Benzoyloxy-2,2,6,6-tetramethylpiperidin, 4-Stearoyloxy-2,2,6,6-tetramethylpiperidin, Bis(2,2,6,6-tetramethylpiperidyl)-sebacat, 3-n-Octyl-7,7,9,9-tetramethyl-1,3,8-triazaspiro[4,5]decan-2,4-dion.

*Oxalsäurediamide,* wie z.B. 4,4'-Di-octyloxy-oxanilid, 2,2'-Di-octyloxy-5,5'-di-tert.butyl-oxanilid, 2,2-Didodecyloxy-5,5'-Di-tert.butyl-oxanilid, 2-Aethoxy-2', äthyl-oxanilid, N,N'-Bis-(3-dimethylaminopropyl)-oxalamid, 2-Aethoxy-5-tert.butyl-2'-äthyl-oxanilid und dessen Gemisch mit 2-Aethoxy-2'-äthyl-5,4'-di-tert. butyl-oxanilid, Gemische von ortho- und para-Methoxysowie von o- und p-Aethoxydisubstituierten Oxaniliden.

Das erfindungsgemässe Stoffgemisch gewährleistet eine ausgezeichnete thermostabilisierende Wirkung, welche diejenige der Einzelkomponenten weit übersteigt.

Die nachfolgenden Beistiele dienen der näheren Erläuterung der Erfindung. Teile sind dabei Gewichtsteile und Prozente, Gewichtsprozente.

*Herstellungsbeispiele*

Beispiel A

1,4-Dihydro-2,6-dimethylpyridin-3,5-dicarbonsäure-diäthylester:

Eine Mischung aus

13,0 g (0,1 Mol) Acetessigsäure-äthylester
4,3 g Formalin (entspr. 0.05 Mol Formhaldehyd)
3,9 g (0,05 Mol Ammoniumcarbaminat
85,0 ml Isopropanol
15,0 ml Wasser

wird 20 Minuten lang auf 80°C erhitzt. Nach dem Abkühlen und Einengen des Lösungsmittels kristallisiert der 1,4-Dihydro-2,6-dimethylpyridin-3,5-dicarbonsäure-diäthylester aus.

Ausbeute: 9,9 g (78% d.Th.)

Schmelzpunkt: 182—187°C

| Analyse: | % C | % H | % N |
|---|---|---|---|
| gefunden | 61,7 | 7,7 | 5,6 |
| berechnet | 61,6 | 7,6 | 5,5 |

Beispiel B

1,4-Dihydro-2,6-dimethylpyridin-3,5-dicarbonsäure-di-(2′-äthylthioäthyl)ester:

Verfährt man wie in Beispiel A, mit der einzigen Ausnahme, dass der Acetessigsäure-äthylester mit 19,0 g (0,1 Mol) Acetessigsäure-(2-äthylthioäthyl)ester ersetzt wird, so erhält man den 1,4-Dihydro-2,6-dimethylpyridin-3,5-dicarbonsäure-di-(2′-äthylthioäthyl)ester.

Ausbeute: 14,4 g (77% d.Th.)

Schmelzpunkt: 102—104°C

| Analyse: | % C | % H | % N | %S |
|---|---|---|---|---|
| gefunden | 54,3 | 7,1 | 3,6 | 17,1 |
| berechnet | 54,7 | 7,3 | 3,8 | 17,2 |

Beispiel C

1,4-Dihydro-2,6-dimethylpyridin-3,5-dicarbonsäure-di-(2′-laurylthioäthyl)ester:

Verfährt man wie in Beispiel A, mit der einzigen Ausnahme, dass der Acetessigsäure-äthylester mit 33,1 g (0,1 Mol) Acetessigsäure-(2-laurylthioäthyl)ester ersetzt wird, so erhält man den 1,4-Dihydro-2,6-dimethylpyridin-3,5-dicarbonsäure-di-(2′-laurylthioäthyl)ester.

Ausbeute: 23,2 g (71% d.Th.)

Schmelzpunkt: 92—94°C

| Analyse: | % C | % H | % N | %S |
|---|---|---|---|---|
| gefunden | 68,2 | 10,6 | 2,1 | 10,0 |
| berechnet | 67,9 | 10,3 | 2,1 | 9,8 |

Beispiel D

1,4-Dihydro-2,6-dimethylpyridin-3,5-dicarbonsäure-di(3′-laurylthio-n-propyl)ester:

Verfährt man wie in Beispiel A, mit der einzigen Ausnahme, dass der Acetessigsäure-äthylester mit 20,4 g (0,1 Mol) Acetessigsäure-(3-laurylthio-n-propyl)ester ersetzt wird, so erhält man den 1,4-Dihydro-2,6-dimethylpyridin-3,5-dicarbonsäure-di-(3′-laurylthio-n-propyl)ester.

Ausbeute: 24,2 g (70% d.Th.)

Schmelzpunkt: 87—90°C

| Analyse: | % C | % H | % N | %S |
|---|---|---|---|---|
| gefunden | 68,2 | 10,0 | 2,2 | 9,2 |
| berechnet | 68,7 | 10,5 | 2,1 | 9,4 |

Beispiel E

1,4-Dihydro-2,6-dimethylpyridin-3,5-dicarbonsäure-di-(2'-äthoxyäthyl)ester:

Verfährt man wie in Beispiel A, mit der einzigen Ausnahme, dass der Acetessigsäure-äthylester mit 17,4 g (0,1 Mol) Acetessigsäure-(2-äthoxyäthyl)ester ersetzt wird, so erhält man den 1,4-Dihydro-2,6-dimethylpyridin-3,5-dicarbonsäure-di(2'-äthoxyäthyl)ester.

Ausbeute: 12,7 g (68% d.Th.)

Schmelzpunkt: 97—100°C

| Analyse: | % C | % H | % N |
|---|---|---|---|
| gefunden | 59,5 | 8,0 | 4,0 |
| berechnet | 59,8 | 8,0 | 4,1 |

*Applikationsbeispiele*

*Prüfung auf Thermostabilität*

Eine Trockenmischung bestehend aus 100 Teilen S—PVC (K-Wert 58), 4 Teilen epoxidiertem Sojabohnenöl, 0,2 Teilen Montanwachs, 5 Teilen MBS* (modifizierungsmittel), 2 Teile PMMA* (Verarbeitungshilfe) und der in der nachstehenden Tabelle in % (bezogen auf die Gesamtzusammensetzung) angegebenen Menge an Dihydropyridindicarbonsäureester und metallhaltigem Stabilisator wird auf einem Mischwalzwerk 5 Minuten bei 190°C gewalzt. Von dem gebildeten Walzfell werden Testfolienstücke von 0,3 mm Dicke entnommen.

Die Folienproben werden in einem Ofen bei 180°C thermisch belastet und alle 3 Minuten an einer Probe der Yellowness-Index (YI) nach ASTM D 1925—70 bestimmt.

Die Ergebnisse sind in der nachstehenden Tabelle zusammengefasst.

---

* MBS = Methacrylsäureester/Butadien/styrol-Mischpolymerisat
* PMMA = Polymethylmethacrylat

| Test Nr. | Testsubstanzen | | | | Yellowness Index n. ASTM D 1925–70 und Belastungszeit KMin.) | | | | | | | | | | | | | | |
|---|---|---|---|---|---|---|---|---|---|---|---|---|---|---|---|---|---|---|---|
| | Dihydropyridindicarbonsäureester | %* | Metallhaltiger Stabilisator | %* | 3 Min. | 6 Min. | 9 Min. | 12 Min. | 15 Min. | 18 Min. | 21 Min. | 24 Min. | 27 Min. | 30 Min. | 33 Min. | 36 Min. | 39 Min. | 42 Min. | 45 Min. |
| 1 | – | – | – | – | 44 | >100 | | | | | | | | | | | | | |
| 2 | Beispiel A | 1,5 | – | – | 11 | 17 | 33 | 63 | 87 | >100 | | | | | | | | | |
| 3 | Beispiel B | 1,5 | – | – | 14 | 20 | 29 | 46 | 67 | 94 | >100 | | | | | | | | |
| 4 | – | – | BCM* | 0,5 | 12 | 23 | 42 | 87 | >100 | | | | | | | | | | |
| 5 | Beispiel A | 1,5 | BCM | 0,5 | 11 | 11 | 11 | 11 | 13 | 22 | 34 | 87 | >100 | | | | | | |
| 6 | Beispiel B | 1,5 | BCM | 0,5 | 12 | 13 | 13 | 14 | 18 | 23 | 31 | 54 | >100 | | | | | | |
| 7 | – | – | MTS* | 0,2 | 13 | 22 | 44 | 62 | 75 | 95 | >100 | | | | | | | | |
| 8 | Beispiel A | 1,5 | MTS | 0,2 | 12 | 13 | 14 | 16 | 18 | 22 | 27 | 33 | 47 | 59 | 92 | >100 | | | |
| 9 | Beispiel B | 1,5 | MTS | 0,2 | 13 | 16 | 17 | 18 | 19 | 21 | 23 | 24 | 25 | 29 | 32 | 42 | 57 | >100 | |
| 10 | – | – | BTS* | 0,2 | 6 | 10 | 16 | 20 | 29 | 39 | 49 | 62 | 69 | 77 | 88 | 91 | 98 | >100 | |
| 11 | Beispiel A | 1,5 | BTS | 0,2 | 11 | 11 | 11 | 11 | 15 | 17 | 19 | 25 | 31 | 46 | 70 | 88 | >100 | | |
| 12 | Beispiel B | 1,5 | BTS | 0,2 | 12 | 13 | 13 | 15 | 16 | 19 | 21 | 23 | 27 | 29 | 37 | 45 | 66 | 79 | 90 |
| 13 | – | – | DBTM* | 0,2 | 37 | 86 | >100 | | | | | | | | | | | | |
| 14 | Beispiel A | 1,5 | DBTM | 0,2 | 12 | 13 | 17 | 23 | 27 | 34 | 57 | 91 | >100 | | | | | | |
| 15 | Beispiel B | 1,5 | DBTM | 0,2 | 14 | 17 | 20 | 26 | 32 | 42 | 53 | 66 | 87 | >100 | | | | | |
| 16 | Beispiel E | 1,5 | – | – | 19 | 34 | 67 | 100 | | | | | | | | | | | |
| 17 | Beispiel E | 1,5 | MTS | 0,2 | 13 | 13 | 15 | 17 | 21 | 25 | 31 | 36 | 41 | 48 | | | | | |

* % = bezogen auf die Gesamtzusammensetzung

* BCM = pulverförmiger Stabilisator, Mischung aus Baund Cd-Myristinat

* BTS = Butylthiostannonsäure $(C_4H_9SnS_{1,5})_n$

* MTS = Monobutylzinnstatilisator der Formel

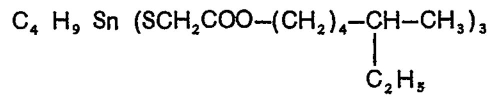

* DBTM = Dibutylzinnmaleinat

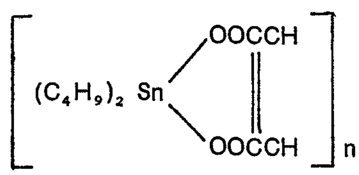

**Patentansprüche**

1. Stoffgemisch bestehend aus
a) einem Dicarbonsäurediester der Formal (I)

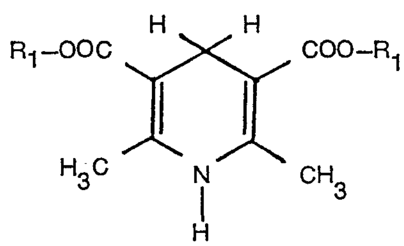

(I)

worin $R_1$ verzweigtes oder unverzweigtes $C_1$—$C_{20}$ Alkyl, $C_5$—$C_7$ Cycloalkyl, Phenyl oder eine Gruppe der Formel $—(CH_2)_p—K—R$ ist, worin x Schwefel oder Sauerstoff, R verzweigtes oder unverzweigtes $C_1$—$C_{20}$ Alkyl und
p die Zahlen 1—8 bedeuten, und
b) einem Metall aus der Gruppe Barium, Cadmium, Zinn oder Antimon oder ferner auch Zink kombiniert mit mindestens einen der vorgenannten Metalle enthaltenden metallhaltigen Stabilisatoren.

2. Stoffgemisch gemäss Anspruch 1, dadurch gekennzeichnet, dass ein Dicarbonsäurediester der Formel (I) verwendet wird, worin $R_1$ eine Gruppe der Formel

$$—(CH_2)_p—X—R$$

ist, worin X, R und p die in Anspruch 1 angegebene Bedeutung haben.

3. Stoffgemisch gemäss Anspruch 1, dadurch gekennzeichnet, dass ein Dicarbonsäurediester der Formel (I) verwendet wird, worin $R_1$ eine Gruppe der Formel

$$—(CH_2)_p—S—(CH_2)_q—CH_3$$

ist, worin p die Zahlen 1—4 und q die Zahlen 1—11 bedeuten.

4. Stoffgemisch gemäss Anspruch 1, dadurch gekennzeichnet, dass ein Dicarbonsäurediester der Formel (I) verwendet wird, worin $R_1$ eine Gruppe der Formel

$$—(CH_2)_p—S—(CH_2)_{11}—CH_3$$

ist, worin p die Zahlen 2 oder 3 bedeutet.

5. Stoffgemisch gemäss den Ansprüchen 1—4, dadurch gekennzeichnet, dass man als metallhaltigen Stabilisator einen Ba/Zn— oder Ba/Cd/Zn-Stabilisator verwendet.

6. Stoffgemisch gemäss den Ansprüchen 1—4, dadurch gekennzeichnet, dass man als metallhaltigen Stabilisator einen Organozinnstabilisator verwendet.

7. Verbindung der Formel II

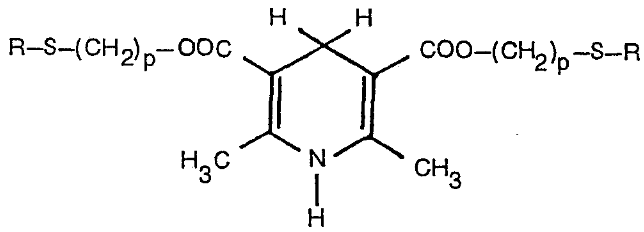

(II)

worin R verzweigtes oder unverzweigtes $C_1$—$C_{20}$ Alkyl und p die Zahlen 1—8 bedeuten.

8. Verbindung gemäss Anspruch 7, der Formel (III)

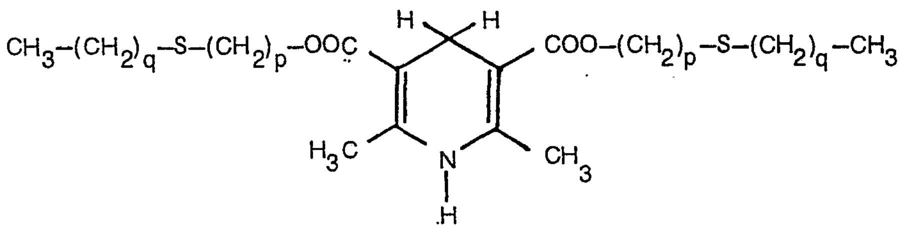

(III)

worin p die Zahlen 1—4, und q die Zahlen 1—11 bedeuten.

9. Verbindung gemäss Anspruch 8 der Formel (III), worin p die Zahlen 2 oder 3 und q die Zahl 11 bedeuten.

10. Stabilisierte Zusammensetzung enthaltend ein chlorhaltiges thermoplastisches Polymer und als Stabilisator ein Stabilisatorgemisch gemäss Anspruch 1 bestehend aus 0,5—2,5 Gew.-% des Dicarbonsäurediesters der Formel (I) und 0,05—1,5 Gew.-% des metallhaltigen Stabilisators, bezogen auf die gesamte Zusammensetzung.

11. Stabilisierte Zusammensetzung gemäss Anspruch 10, dadurch gekennzeichnet, dass sie zusätzlich einen Lichstabilisator enthält.

12. Stabilisierte Zusammensetzung gemäss Anspruch 10 und 11 dadurch gekennzeichnet, dass der chlorhaltige Thermoplast ein Polymer aus oder auf der Grundlage von Vinylchlorid ist.

13. Stabilisierte Zusammensetzung gemäss Anspruch 12, dadurch gekennzeichnet, dass die Mischung zusätzlich einen Weichmacher enthält.

14. Stabilisierte Zusammensetzung gemäss Anspruch 12, dadurch gekennzeichnet, dass der chlorhaltige Thermoplast ein Hart-PVC ist.

**Revendications**

1. Mélange constitué:

a) d'un diester d'acide dicarboxylique répondant à la formule I

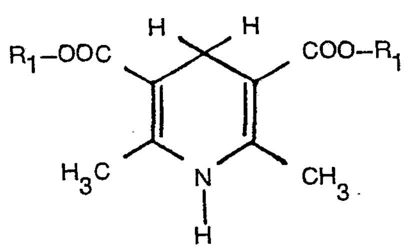

(I)

dans laquelle $R_1$ représente un radical alkyle en $C_1$—$C_{20}$, ramifié ou non, un radical cyclo-alkyle en $C_5$—$C_7$, un radical phényle ou un radical —$(CH_2)_p$—X—R dans lequel X représente le soufre ou l'oxygene, R représente un radical alkyle en $C_1$—$C_{20}$, ramifié ou non, et p désigne un nombre de 1 à 8, et b) d'un stabilisant contenant un métal pris dans l'ensemble constitué par le baryum, le cadmium, l'étain, l'antimoine, et également le zinc associé à au moins l'un des métaux qui viennent d'être cités.

2. Mélange selon la revendication 1, caractérisé en ce qu'il contient un diester d'acide dicarboxylique de formule 1 dans lequel $R_1$ représente un radical:

$$—(CH_2)_p—X—R$$

dans lequel X, R et p ont les significations données à la revendication 1.

3. Mélange selon la revendication 1, caractérisé en ce qu'il contient un diester d'acide dicarboxylique de formule I dans lequel $R_1$ représente un radical:

$$-(CH_2)_p-S-(CH_2)_q-CH_3$$

dans lequel p désigne un nombre de 1 à 4 et q un nombre de 1 à 11.

4. Mélange selon la revendication 1, caractérisé en ce qu'il contient un diester d'acide dicarboxylique de formule I dans lequel $R_1$ représente un radical:

$$-(CH_2)_p-S-(CH_2)_{11}-CH_3$$

dans lequel p désigne le nombre 2 ou le nombre 3.

5. Mélange selon l'une quelconque des revendication 1 à 4, caractérisé en ce qu'il contient, comme stabilisant métallisé, un stabilisant contenant Ba/zn ou Ba/Cd/Zn.

6. Mélange selon l'une quelconque des revendications 1 à 4, caractérisé en ce qu'il contient, comme stabilisant métallisé, un stabilisant organostannique.

7. Composé répondant à la formule II

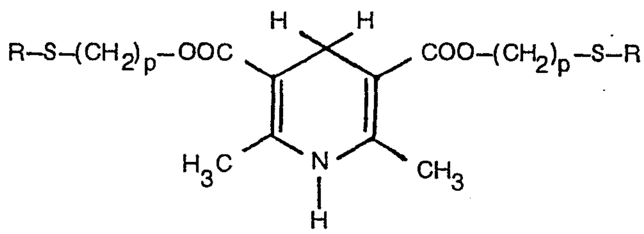

(II)

dans laquelle R représente un radical alkyle, linéaire ou ramifié, contenant de 1 à 20 atomes de carbone et p désigne un nombre de 1 à 8.

8. Composé selon la revendication 7, qui répond à la formule III

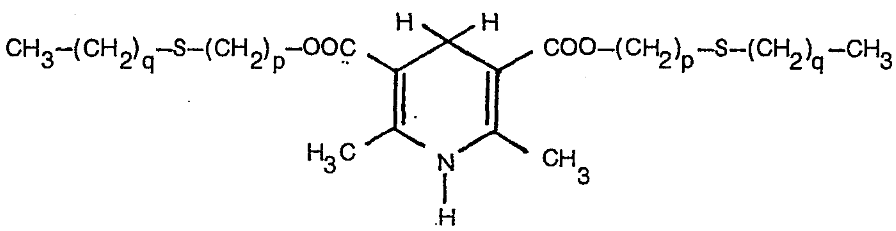

(III)

dans laquelle p désigne un nombre de 1 à 4 et q un nombre de 1 à 11.

9. Composé de formule III selon la revendication 8 dans lequel p désigne le nombre 2 ou le nombre 3 et q le nombre 11.

10. Composition stabilisée contenant un polymère thermoplastique chloré et, comme stabilisant, un mélange stabilisant selon la revendication 1 constitué de 0,5 à 2,5% en poids d'un diester d'acide dicarboxylique de formule I et de 0,05 à 1,5% en poids du stabilisant métallisé, par rapport à la composition totale.

11. Composition stabilisée selon la revendication 10, caractérisée en ce qu'elle contient en outre un stabilisant à la lumière.

12. Composition stabilisée selon l'une des revendications 10 et 11, caractérisée en ce que la matière thermoplastique chlorée est un polymère constitué ou à base de chlorure de vinyle.

13. Composition stabilisée selon la revendication 12, caractérisée en ce que le mélange contient en outre un plastifiant.

14. Composition stabilisée selon la revendication 12, caractérisée en ce que la matière thermoplastique chlorée est un polychlorure de vinyle dur.

## Claims

1. A stabiliser mixture consisting of
a) a dicarboxylic acid diester of the formula (I)

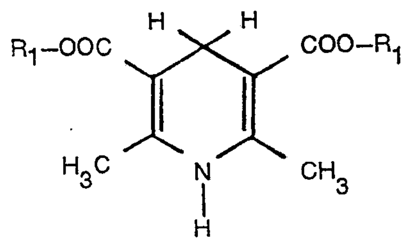

(I)

wherein $R_1$ is branched-chain or straight-chain $C_1$—$C_{20}$-alkyl, $C_5$—$C_7$-cycloalkyl, phenyl, or a group of the formula $-(CH_2)_p-X-R$, wherein X is sulfur or oxygen, R is branched-chain or straight-chain $C_1$—$C_{20}$-alkyl, and p is a number from 1—8, and
b) a metal-containing stabiliser containing a metal from the group comprising barium, cadmium, tin or antimony, or also zinc combined with at least one of the aforementioned metals.

2. A mixture according to Claim 1, in which there is used a dicarboxylic acid diester of the formula (I) wherein $R_1$ is a group of the formula

$$-(CH_2)_p-X-R$$

wherein X, R and p have the meaning defined in Claim 1.

3. A mixture according to Claim 1, in which there is used a dicarboxylic acid diester of the formula (I) wherein $R_1$ is a group of the formula

$$-(CH_2)_p-S-(CH_2)_q-CH_3$$

wherein p is a number from 1—4, and q a number from 1—11.

4. A mixture according to Claim 1, in which there is used a dicarboxylic acid diester of the formula (I) wherein $R_1$ is a group of the formula

$$-(CH_2)_p-S-(CH_2)_{11}-CH_3$$

wherein p is the number 2 or 3.

5. A mixture according to Claims 1—4, wherein the metal-containing stabiliser used is a Ba/Zn or Ba/Cd/Zn stabiliser.

6. A mixture according to Claims 1—4, wherein the metal-containing stabiliser used is an organotin stabiliser.

7. A compound of the formula II

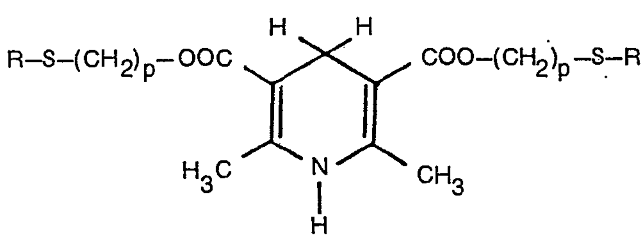

(II)

wherein R is branched-chain or straight-chain $C_1$—$C_{20}$ alkyl, and p is a number from 1—8.

8. A compound according to Claim 7 of the formula (III)

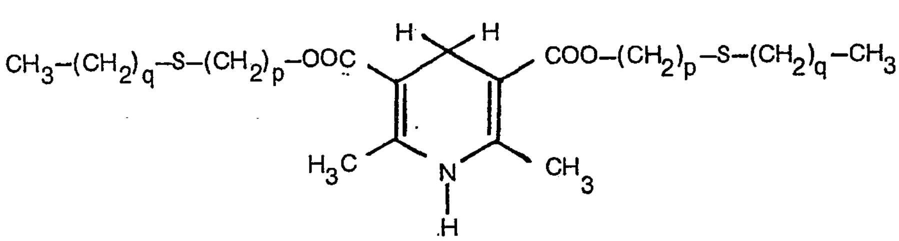

(III)

wherein p is a number from 1—4 and q a number from 1—11.

9. A compound according to Claim 8 of the formula (III), wherein p is the number 2 or 3, and q is the number 11.

10. A stabilised composition containing a chlorine-containing thermoplastic polymer and, as stabiliser, a stabiliser mixture according to Claim 1 consisting of 0.5—2.5 per cent by weight of the dicarboxylic acid diester of the formula (I) and 0.05—1.5 per cent by weight of the metal-containing stabiliser, relative to the total composition.

11. A stabilised composition according to Claim 10, which additionally contains a light stabiliser.

12. A stabilised composition according to Claims 10 and 11, wherein the chlorine-containing thermoplast is a polymer formed from or based on vinyl chloride.

13. A stabilised composition according to Claim 12, wherein the mixture additionally contains a plasticiser.

14. A stabilised composition according to Claim 12, wherein the chlorine-containing thermoplast is a rigid PVC.